# EUROPEAN PATENT APPLICATION

(11) **EP 3 633 276 A1**
(43) Date of publication of application: **08.04.2020**
(21) Application number: 19201505.5
(22) Date of filing: 04.10.2019
(51) Int. Cl.: F24F 3/16, B64D 13/00, F24F 7/06, A61L 2/10

(54) **AIR PURIFIER SYSTEM WITH ULTRAVIOLET LIGHT ASSEMBLY**

(30) Priority: 05.10.2018 US 201816153344
(71) Applicant: Hamilton Sundstrand Corporation, Charlotte, NC 28217-4578 (US)
(72) Inventor: RHODEN, William E., Glastonbury, CT Connecticut 06033 (US)
(74) Representative: Dehns

(57) **Abstract**

An air purifier system includes a duct (28), an ultraviolet light assembly (30), and a fan (32). The duct defines a reaction chamber (46) for the flow of air (26), and includes an inlet portion (40) and an outlet portion (42). The ultraviolet light assembly is adapted to illuminate the reaction chamber with ultraviolet light. The fan is adapted to draw the air through the reaction chamber. A reflective surface (54) of the fan is configured to reflect the ultraviolet light into the reaction chamber.

## Description

### BACKGROUND

The present disclosure relates to air purifier systems, and more particularly to air purifier systems constructed to purify air of a cabin and utilizing an ultraviolet light assembly.

Air quality is of significant concern when exposed to occupants within dwellings and transportation vehicles. Of particular concern is the recirculation of airborne pathogens. The use of ultraviolet light to destroy such pathogens is generally known. However, the efficiency of such systems requires undesirably, relatively large, reaction chambers and/or relatively high energy use. Improving the efficiency and reducing the size of air purifier systems using ultraviolet light technology is desirable.

### BRIEF DESCRIPTION

An air purifier system according to one aspect of the present disclosure comprises a duct defining a reaction chamber for the flow of air through the duct, the duct including an inlet portion and an outlet portion; an ultraviolet light assembly adapted to illuminate the reaction chamber with ultraviolet light; and a fan in operable communication with the reaction chamber and exposed to the air flowing through the reaction chamber, the fan including a reflective surface positioned and configured to reflect the ultraviolet light impinging upon the reflective surface.

In accordance with an embodiment, the fan may be adapted to draw the air through the reaction chamber, and be located at the outlet portion and the reflective surface faces at least in part upstream with respect to the flow of air.

In the alternative or additionally thereto, in the foregoing embodiment, the air purifier system may comprise a pre-filter disposed at the inlet portion.

In the alternative or additionally thereto, in the foregoing embodiment, the duct may include a reflective face defining the reaction chamber.

In the alternative or additionally thereto, in the foregoing embodiment, the ultraviolet light may be within a UV-C range.

In the alternative or additionally thereto, in the foregoing embodiment, the ultraviolet light may be within a wavelength range of about 185 nanometers to 254 nanometers.

In the alternative or additionally thereto, in the foregoing embodiment, the ultraviolet light may be within a wavelength range of about 220 nanometers to 290 nanometers.

In the alternative or additionally thereto, in the foregoing embodiment, the air purifier system may further comprise a louver disposed at the inlet portion and adapted to swirl the flow of air within the reaction chamber.

In the alternative or additionally thereto, in the foregoing embodiment, the reflective surface may be polished aluminum.

In the alternative or additionally thereto, in the foregoing embodiment, the reflective surface may be polished stainless steel.

In the alternative or additionally thereto, in the foregoing embodiment, the ultraviolet light assembly may be disposed in the reaction chamber and may be adapted to direct the ultraviolet light axially toward the fan.

In the alternative or additionally thereto, in the foregoing embodiment, the ultraviolet light assembly may include a ballast centered to the centerline and an ultraviolet light source projecting outward from the ballast and in a downstream direction toward the fan.

A vehicle according to another aspect includes a cabin adapted to be occupied; and an air purifier system adapted to kill pathogens from air circulating through the cabin. The air purifier system includes a duct defining a reaction chamber for the flow of the air, the duct including an inlet portion and an outlet portion, an ultraviolet light assembly adapted to illuminate the reaction chamber with ultraviolet light, and a fan adapted to draw the air through the reaction chamber, the fan including a reflective surface facing the ultraviolet light assembly for reflecting the ultraviolet light back into the reaction chamber.

In addition to the foregoing embodiment, the vehicle may be an aircraft.

In the alternative or additionally thereto, in the foregoing embodiment, the vehicle may further comprise a pre-filter disposed at the inlet portion upstream of the reaction chamber, wherein the pre-filter is adapted to remove particulate from the air prior to entering the reaction chamber.

In the alternative or additionally thereto, in the foregoing embodiment, the ultraviolet light may be within a wavelength range of about 220 nanometers to 290 nanometers.

In the alternative or additionally thereto, in the foregoing embodiment, the reflective surface may be polished aluminum.

In the alternative or additionally thereto, in the foregoing embodiment, the reflective surface may be polished stainless steel.

In the alternative or additionally thereto, in the foregoing embodiment, the ultraviolet light assembly may be disposed in the reaction chamber, and may include a ballast centered to the centerline and an ultraviolet light source projecting outward from the ballast and in a downstream direction toward the fan.

A method of killing pathogens in a vehicle according to another aspect, comprises drawing air from a cabin of a vehicle with a fan; directing the air through a reaction chamber; and reflecting ultraviolet light off of a plurality of reflective surfaces of the fan and into the reaction chamber.

The foregoing features and elements may be combined in various configurations without exclusivity, unless expressly indicated otherwise. These features and elements as well as the operation thereof will become more apparent in light of the following description and the accompanying drawings. However, it should be understood that the following description and drawings are intended to be exemplary in nature and non-limiting.

### BRIEF DESCRIPTION OF THE DRAWINGS

Various features will become apparent to those skilled in the art from the following detailed description of the disclosed non-limiting embodiments. The drawings that accompany the detailed description can be briefly described as follows:
FIG. 1 is a schematic of a vehicle having a cabin that utilizes an air purifier system to purify air recirculated through the cabin; and
FIG. 2 is a schematic of the air purifier system.

### DETAILED DESCRIPTION

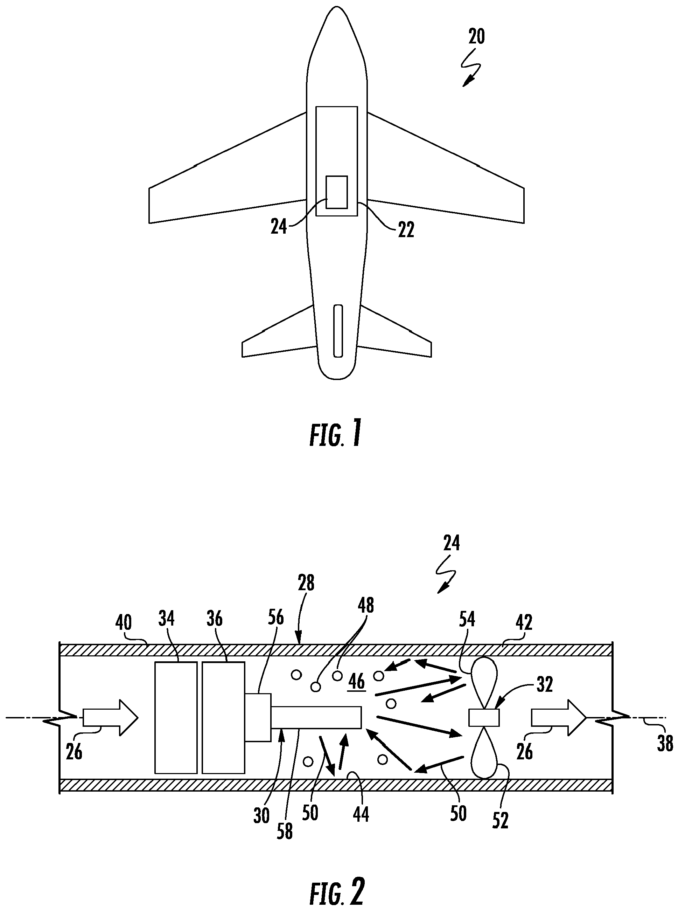

Referring to FIG. 1, a vehicle 20 includes a cabin 22 that contains air which is purified by an air purifier system 24. The cabin 22 may be constructed to be occupied by, for example, humans. Use of the air purifier system 24 is particularly useful in applications where the cabin 22 environment is isolated from external environments. For example, a cabin may be pressurized with respect to the outside ambient atmosphere, or the cabin air may be temperature controlled and at a temperature different than the outside ambient air temperature. As one, non-limiting, application, the vehicle 20 is illustrated as an aircraft, or airplane. However, the vehicle 20 may be any other type of vehicle that includes the cabin 22. Examples may include, but are not limited to, trains, automobiles, marine crafts, and others. It is further contemplated and understood that the air purifier system 24 may also be applied to non-transportation applications, including dwellings and other structures having occupied spaces or rooms.

Referring to FIG. 2, the air purifier system 24 is adapted to draw air (see arrows 26) from the cabin 22, purify the air 26, and flow the air back into the cabin 22. Purification of the air 26 at least includes the destruction of airborne pathogens. It is contemplated and understood that the air purifier system 24 may be independent from other air conditioning systems, or may be an integral part of such system. For example, the air purifier system 24 may be an integral part of a heating, ventilation, and air conditioning (HVAC) system also known to control air temperature and/or air humidity. It is further contemplated that the air purifier system 24 may be installed as a unit into pre-existing duct work of the HVAC system.

The air purifier system 24 may include a duct 28, an ultraviolet light assembly 30, a fan 32, a filter 34, and a louver 36. The duct 28 generally extends along an axis 38 (i.e., centerline), and extends axially between an inlet portion 40 and an outlet portion 42 of the duct 28. During operation, the air 26 (in an unpurified state) flows from the cabin 22, into the inlet portion 40, and axially out through the outlet portion 42 (in a purified state) for flow back into the cabin 22.

The duct includes an internal face 44 that may be circumferentially continuous, faces radially inward, and defines a reaction chamber 46 axially located between the inlet and outlet portions 40, 42. In one example, the ultraviolet light assembly 30 is located in the reaction chamber 46. The filter 34 is located at the inlet portion 40 and upstream from the reaction chamber 46. The louver 36 is located at the inlet portion 40 and downstream from the filter 34. The fan 32 is located at the outlet portion 42 downstream from the reaction chamber 46.

The filter 34 is adapted to reduce, or eliminate the flow of particulate in the air 26, and before the air reaches the reaction chamber 46. In one example, the louver 36 is adapted to divert the flow pattern of the incoming air 26 to that of a spiraling pattern for optimizing the performance of the ultraviolet light assembly 30 in the destruction of pathogens 48 within the reaction chamber 46. It is contemplated and understood that the filter 34 may be located at the outlet portion 42 of the duct 28, or an additional filter may be located at the outlet portion.

In one example, the face 44 may be reflective for the reflection of ultraviolet light emitted by the ultraviolet light assembly 30 (i.e., energy or rays, see arrows 50) back into the reaction chamber 46 to optimize performance of the ultraviolet light assembly 30 in the destruction of the pathogens 48. Similarly, each airfoil 52 of the fan 32 may include a reflective surface 54 that faces, at least in-part toward the reaction chamber 46. In operation, ultraviolet rays 50 reflect off of the angled, reflective surfaces 54 of each airfoil 52 and back into the reaction chamber 46 at various angles for improved distribution of the ultraviolet rays 50 for improved destruction of the pathogens 48. The reflective face 44 and the reflective surfaces 54 may be made of polished aluminum, polished stainless steel, or any other material or composition capable of reflected ultraviolet rays 50. In another example, the reflective face 44 may be a diffused reflector that reflects light at all angles to expose all air contaminant molecules (i.e., pathogens) from all sides.

The ultraviolet light assembly 30 may include a ballast 56 and an ultraviolet light source 58. In one example, the ultraviolet light source 58 emits the ultraviolet light 50 within a wavelength range of about 220 nanometers to 290 nanometers, and/or at a UV-C energy. In another embodiment, the wavelength range may be about 185 nanometers to 254 nanometers. In this example, the ultraviolet light assembly 30 also facilitates the production of ozone at about 185 nanometers, and destroys pathogens (i.e., germicidal) at about 254 nanometers.

In one embodiment, the ballast 56 of the ultraviolet light assembly 30 is located in the reaction chamber 46, is proximate to the inlet portion 40 and/or louver 36, and is centered to the centerline 38. The ultraviolet light source 58 is located in the reaction chamber 46, axially projects from the ballast 56 and toward the fan 32, and is substantially centered to the centerline 38 for efficient performance. It is contemplated and understood that the ballast 56 and/or the ultraviolet light source 58 may be orientated elsewhere, and may be located outside of the reaction chamber 46 provided the ultraviolet light 50 is efficiently emitted into the reaction chamber 46.

In one embodiment, the fan 32 may be a passive fan employed to rotate (i.e., like windmill) via the air flowing through it. An electrically driven fan may be located elsewhere in the system, not exposed to the ultraviolet light, and to induce the flow of air.

Advantages and benefits of the present disclosure include an air purifier system 20 capable of efficiently destroying pathogens while minimizing the size of a reaction chamber 46 and/or reducing the needed wattage for operation of the ultraviolet light assembly 30

While the present disclosure is described with reference to exemplary embodiments, it will be understood by those skilled in the art that various changes may be made and equivalents may be substituted without departing from the scope of the invention as defined by the claims. In addition, various modifications may be applied to adapt the teachings of the present disclosure to particular situations, applications, and/or materials, without departing from the scope of the claims. The present disclosure is thus not limited to the particular examples disclosed herein, but includes all embodiments falling within the scope of the appended claims.

## Claims

1. An air purifier system comprising:
a duct (28) defining a reaction chamber (46) for the flow of air (26) through the duct, the duct including an inlet portion (40) and an outlet portion (47);
an ultraviolet light assembly (30) adapted to illuminate the reaction chamber with ultraviolet light; and
a fan (32) in operable communication with the reaction chamber and exposed to the air flowing through the reaction chamber, the fan including a reflective surface (54) positioned and configured to reflect the ultraviolet light impinging upon the reflective surface.

2. The air purifier system set forth in claim 1, wherein the fan is adapted to draw the air through the reaction chamber, and is located at the outlet portion and the reflective surface (54) faces at least in part upstream with respect to the flow of air.

3. The air purifier system set forth in claim 2, further comprising:
a pre-filter (34) disposed at the inlet portion.

4. The air purifier system set forth in claim 2 or 3, wherein the duct includes a reflective face (44) defining the reaction chamber.

5. The air purifier system set forth in claim 2, 3 or 4, wherein the ultraviolet light is within a UV-C range.

6. The air purifier system set forth in any of claims 1 to 4, wherein the ultraviolet light is within a wavelength range of about 185 nanometers to 254 nanometers.

7. The air purifier system set forth in any of claims 1 to 4, wherein the ultraviolet light is within a wavelength range of about 220 nanometers to 290 nanometers.

8. The air purifier system set forth in any preceding claim, further comprising a louver (36) disposed at the inlet portion and adapted to swirl the flow of air within the reaction chamber.

9. The air purifier system set forth in any preceding claim, wherein the reflective surface is polished aluminum.

10. The air purifier system set forth in any preceding claim, wherein the reflective surface is polished stainless steel.

11. The air purifier system set forth in any preceding claim, wherein the ultraviolet light assembly (30) is disposed in the reaction chamber (46) and is adapted to direct the ultraviolet light axially toward the fan.

12. The air purifier system set forth in claim 11, wherein the ultraviolet light assembly (30) includes a ballast (56) centered to the centerline and an ultraviolet light source projecting outward from the ballast and in a downstream direction toward the fan.

13. A vehicle comprising:
a cabin (22) adapted to be occupied; and
an air purifier system (24) as claimed in any preceding claim, adapted to kill pathogens (48) from air circulating through the cabin.

14. The vehicle set forth in claim 12 or 13, wherein the vehicle is an aircraft.

15. A method of killing pathogens (48) in a vehicle comprising:
drawing air from a cabin (22) of a vehicle with a fan (32);
directing the air through a reaction chamber (46); and
reflecting ultraviolet light off of a plurality of reflective surfaces (54) of the fan and into the reaction chamber.
